(19) Europäisches Patentamt
European Patent Office
Office européen des brevets

(11) Publication number: 0 195 462 B1

(12) EUROPEAN PATENT SPECIFICATION

(45) Date of publication of patent specification: 02.05.90

(51) Int. Cl.$^5$: C 10 L 1/22

(21) Application number: 86105869.1

(22) Date of filing: 16.08.83

(60) Publication number of the earlier application in accordance with Art. 76 EPC: 0 101 093

# (54) Diesel fuel composition.

(30) Priority: 16.08.82 US 408074
27.09.82 US 423606
27.09.82 US 424054
27.09.82 US 424053
06.10.82 US 433160
15.10.82 US 434632
09.11.82 US 440182

(43) Date of publication of application:
24.09.86 Bulletin 86/39

(45) Publication of the grant of the patent:
02.05.90 Bulletin 90/18

(84) Designated Contracting States:
AT BE CH DE FR GB IT LI LU NL SE

(56) References cited:
FR-A-2 511 390

(73) Proprietor: ETHYL CORPORATION
Ethyl Tower 451 Florida Boulevard
Baton Rouge Louisiana 70801 (US)

(72) Inventor: Filbey, Allen Howard
13443 Anne Cleeves Avenue
Baton Rouge Louisiana 70816 (US)
Inventor: Hinkamp, James Benjamin
1444 South Bates Street
Birmingham Michigan 48009 (US)
Inventor: Seemuth, Paul Douglas
7623 Oakmount Drive
Baton Rouge Louisiana 70816 (US)
Inventor: Thomas, Samuel Gabriel, Jr.
16459 Chadsford Avenue
Baton Rouge Louisiana 70817 (US)

(74) Representative: Dipl.-Ing. Schwabe, Dr. Dr. Sandmair, Dr. Marx
Stuntzstrasse 16
D-8000 München 80 (DE)

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

EP 0 195 462 B1

**Description**

Diesel engines operate by compression ignition. They have compression ratios in the range of 14:1 to 17:1 or higher and for that reason obtain more useful work from a given amount of fuel compared to an Otto cycle engine. Historically, diesel engines have been operated on a petroleum-derived liquid hydrocarbon fuel boiling in the range of about 300—750°F (149—399°C). Recently, because of dwindling petroleum reserves, alcohol and alcohol-hydrocarbon blends have been studied for use as diesel fuel.

One major factor in diesel fuel quality is cetane number. Cetane number is related to ignition delay after the fuel is injected into the combustion chamber. If ignition delays too long, the amount of fuel in the chamber increases and upon ignition results in a rough running engine and increased smoke. A short ignition delay results in smooth engine operation and decreases smoke. Commercial petroleum diesel fuels generally have a cetane number of about 40—55. Alcohols have a much lower cetane value and require the addition of a cetane improver for successful engine operation.

Through the years, many types of additives have been used to raise the cetane number of diesel fuel. These include peroxides, nitrites, nitrates, nitroso-carbamates, and the like. Alkyl nitrates such as amyl nitrate, hexyl nitrate and mixed octyl nitrates have been used commercially with good results.

Secondary nitrate esters having nitro substitution are disclosed in U.S. 3,282,983. They are made by reaction in an olefin with dinitrogen tetroxide and oxygen followed by reduction of the intermediate peroxynitrate. According to the disclosure, it is not possible to make a primary nitrate by this method.

British 580,260 and British 586,022, as well as British 604,360, U.S. 2,453,942 and U.S. 2,472,550, are similar and disclose secondary nitrate esters.

The present invention provides an increased cetane rating for a diesel fuel, either hydrocarbon, alcohol or mixed, by the addition of a small but effective amount of a heterocyclic aliphatic nitrate or dinitrate compound.

More specifically the invention relates to a liquid fuel adapted for use in a diesel engine, said fuel being selected from the group consisting of liquid hydrocarbons of the diesel boiling range, alcohols and mixtures thereof, characterized in that said nitrate compound is tetrahydropyranol nitrate.

Representative examples of these additives include:

4-methyltetrahydro-2H-pyran-3-ol nitrate
4-ethyltetrahydro-2H-pyran-3-ol nitrate
tetrahydropyran-2-ol nitrate
3-ethyltetrahydro-3H-pyran-4-ol nitrate
3-dodecyltetrahydro-3H-pyran-4-ol nitrate
3-methyl-tetrahydropyran-2-ol nitrate
5-chlorotetrahydro-2H-pyran-3-ol nitrate
4-bromotetrahydro-2H-pyran-3-ol nitrate
4-methyl-5-chlorotetrahydro-2H-pyran-3-ol nitrate,

and the like. A more preferred class of pyranol nitrates include compounds having the structure:

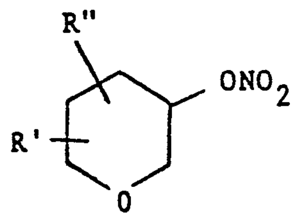

wherein R′ and R″ are independently selected from the group consisting of hydrogen, alkyl containing 1—20 carbon atoms, cycloalkyl containing 5—8 carbon atoms, alkenyl containing 2—20 carbon atoms, cycloalkyl containing 5—8 carbon atoms, alkenyl containing 2—20 carbon atoms, aryl containing 6—12 carbons atoms and aralkyl containing 7—12 carbon atoms. The remaining carbon bonds are hydrogen substituted.

Representative examples of this preferred embodiment include:

4,5-dimethyltetrahydro-2H-pyran-3-ol nitrate
4-octadecyltetrahydro-2H-pyran-3-ol nitrate
4-eicosyltetrahydro-2H-pyran-3-ol nitrate
4-allyltetrahydro-2H-pyran-3-ol nitrate
5-hexadecenyltetrahydro-2H-pyran-3-ol nitrate
5-cyclopentyltetrahydro-2H-pyran-3-ol nitrate
5-cyclohexyltetrahydro-2H-pyran-3-ol nitrate
4-cyclooctyltetrahydro-2H-pyran-3-ol nitrate
4-methyl-5-phenyltetrahydro-2H-pyran-3-ol nitrate
5-benzyltetrahydro-2H-pyran-3-ol nitrate
5-(alpha,alpha-dimethylbenzyl)tetrahydro-2H-pyran-3-ol nitrate
5-(4-sec-pentylbenzyl)tetrahydro-2H-pyran-3-ol nitrate, and the like.

The most preferred tetrahydropyranol nitrate cetane improver is tetrahydro-2H-pyran-3-ol nitrate.

The following examples shows the preparation of a tetrahydropyranol nitrate by reaction of the corresponding hydroxy compound with mixed nitric acid-acetic anhydride.

Example 1

In a reaction vessel was placed 50 ml acetic anhydride. While cooling, 14 ml of 90 percent fuming nitric acid was added. Following this, 25 g of tetrahydro-2H-pyran-3-ol was added dropwise at −8 to −10°C over a one hour period. Stirring was continued for 1.5 hours at −10°C. The mixture was then allowed to warm to room temperature and then poured into an ice-water mixture. The organic phase settled and was separated. the organic phase was diluted with methylene chloride and also washed with aqueous $NaHCO_3$ and dried over $MgSO_4$. The solvent was removed under vacuum leaving 24.04 g of tetrahydro-2H-pyran-3-ol nitrate. The structure was confirmed by IR and NMR analysis.

Other tetrahydropyranol nitrates can be made following the above general procedure by substituting other tetrahydropyranols.

Other conventional additives may be included in the diesel fuel including antioxidants, pour point depressants, cold filter plugging inhibitors, detergents, rust inhibitors and the like, including other cetain improvers.

The amount of cetane improver added depends on the type of fuel being used, the initial cetane value, and the amount of cetane number increased desired. Alcohol fuels such as methanol, ethanol, isopropanol, isobutanol, hexanol, and the like, have very low cetane values and large amounts of cetane improvers are required. A useful range in which to operate is about 5—25 weight percent cetane improver.

Blends of alcohol and petroleum-derived diesel fuel have higher cetane values and require less cetane improver. A useful range is about 0.5—10 weight percent.

Petroleum-derived distillate fuel in the diesel boiling range require only small amounts of cetane improver to achieve a significant increase in cetane number. Such fuels without any cetane improver generally have cetane numbers in the range of about 25—60. Cetane numbers in the range of 25—35 are considered low and those in the range of 50—60 are considered top grade diesel fuels. Diesel fuels in the 35—50 mid-range are most common. An object of the invention is to upgrade the low cetane number fuels at least into the mid-range and to increase the cetane value of the mid-range fuels into the upper portion of the mid-range (e.g., 45—50) or even into the premium range above 50. It has been found that highly beneficial results can be achieved using as little as 0.05 weight percent of the present additive. Accordingly, a useful concentration range in petroleum derived diesel fuel is about 0.01—5 weight percent and more preferably about 0.05—0.5 weight percent.

The cetane increase caused by the tetrahydropyranol nitrate additive was measured in comparison with that caused by a commercial cetane improver, isooctyl nitrate, using a standard cetane engine. The fuel used was a blend of 46 cetane diesel fuel and 28 cetane light cycle oil giving a 38 cetane No. diesel fuel. The results at various concentrations of tetrahydro-2H-pyran-3 ol nitrate and isooctyl nitrate are shown in the following table.

| | Isooctyl Nitrate | Tetrahydro-3H-pyran-3-ol Nitrate |
|---|---|---|
| Concentration | CN | CN |
| None | 38 | 38 |
| 0.15 | 41.64, 41.65 | 42.24, 42.38 |

These results show that the new additives are very effective in raising the cetane number of diesel fuel.

## Claims

1. A liquid fuel adapted for use in a diesel engine, said fuel being selected from liquid hydrocarbons of the diesel boiling range, alcohols and mixtures thereof, containing a cetane number increasing amount of an organic nitrate ester, characterized in that said organic nitrate ester is a tetrahydropyranol nitrate.

2. A fuel compositon as claimed in Claim 1 further characterized in that said tetrahydropyranol nitrate has the structure

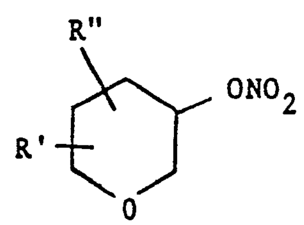

wherein R′ and R″ are independently selected from the group consisting of hdyrogen, alkyl containing 1—20 carbon atoms, cycloalkyl containing 5—8 carbon atoms, alkenyl containing 2—20 carbon atoms, aryl containing 6—12 carbon atoms and aralkyl containing 7—12 carbon atoms.

3. A fuel composition as claimed in Claim 2 further characterized in that said tetrahydropyranol nitrate is the compound tetrahydro-2H-pyran-3-ol nitrate.

**Patentansprüche**

1. Zur Verwendung in einem Dieselmotor angepaßter Kraftstoff, ausgewählt aus flüssigen Kohlenwasserstoffen des Dieselsiedebereichs, Alkoholen und Mischungen davon, enthaltend eine die Cetanzahl erhöhende Menge eines organischen Nitratesters dadurch gekennzeichnet, daß der organische Nitratester ein Tetrahydropyranolnitrat ist.

2. Kraftstoffzusammensetzung nach Anspruch 1, dadurch gekennzeichnet, daß das Tetrahydropyranol die folgende Struktur aufweist:

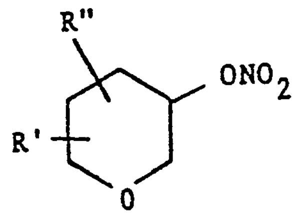

in der R′ und R″ unabhängig voneinander ausgewählt sind uas der Gruppe bestehend aus Wasserstoff, $C_{1-20}$-Alkyl, $C_{5-8}$-Cycloalkyl, $C_{2-20}$-Alkenyl, $C_{6-12}$-Aryl und $C_{7-12}$-Aralkyl.

3. Kraftstoffzusammensetzung nach Anspruch 2, dadurch gekennzeichnet, daß das Tetrahydropyranolnitrat die Verbindung Tetrahydro-2H-pyran-2-ol-nitrat ist.

**Revendications**

1. Carburant liquide adapté à un moteur diesel, le dit carburant étant choisi parmi les hydrocarbures liquides ayant un point d'ébullition dans le domaine du diesel, les alcohols et les mélanges de ceux-ci, contenant une quantité d'un ester nitrate organique propre à élever l'indice de cétane, caractérisé en ce que le dit ester nitrate organique est un nitrate de tétrahydropyranol.

2. Compositon de carburant selon la revendication 1, caractérisé en outre en ce que le dit nitrate de tétrahydropyranol a la structure suivante

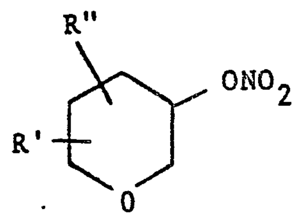

dans laquelle R′ et R″ sont choisis indépendamment dans le groupe de: hydrogène, alkyle contenant 1—20 atomes de carbone, cycloalkyle contenant 5—8 atomes de carbone, alkényle contenant 2—20 atomes de carbone, aryle contenant 6—12 atomes de carbone, et aralkyle contenant 7—12 atomes des carbone.

3. Composition de carburant selon la revendication 2, caractérisé en outre en ce que le dit nitrate de tétrahydropyranol est le nitrate de tétrahydro-2H-pyran-3-ol.